# EUROPÄISCHE PATENTSCHRIFT

(11) **EP 1 261 290 B1**
(45) Veröffentlichungstag und Bekanntmachung des Hinweises auf die Patenterteilung: **26.11.2003**
(21) Anmeldenummer: 02722122.5
(22) Anmeldetag: 22.02.2002
(51) Int. Cl.: A61C 1/00, A61B 18/20

(54) **MEDIZINISCHES LASERBEHANDLUNGSGERÄT**
MEDICAL LASER TREATMENT DEVICE
APPAREIL MEDICAL DE TRAITEMENT AU LASER

(30) Priorität: 22.02.2001 DE 10108655
(43) Veröffentlichungstag der Anmeldung: 04.12.2002
(73) Patentinhaber: W & H Dentalwerk Bürmoos GmbH, 5111 Bürmoos (AT)
(72) Erfinder: BRUGGER, Wilhelm, W & H Dentalwerk Bürmoos GmbH, A-5111 Bürmoos (AT); KASENBACHER, Anton, 83278 Traunstein (DE); NIEMZ, Mark, 76547 Sinzheim (DE); BAUER, Thorsten, 30167 Hannover (DE)
(74) Vertreter: Appelt, Christian W.
(86) Internationale Anmeldenummer: EP0201906
(87) Internationale Veröffentlichungsnummer: WO02067802

(56) Entgegenhaltungen:
- EP-A- 0 284 330
- DE-A- 19 844 719

## Beschreibung

Die vorliegende Erfindung betrifft ein medizinisches Laserbehandlungsgerät mit einer Laserlichtquelle, insbesondere ein zahnärztliches Laserbehandlungsgerät.

Ein solches Laserbehandlungsgerät ist beispielsweise aus der DE 195 33 348 A1 bekannt. Neben der Laserlichtquelle umfaßt ein solches Gerät bevorzugt Bedienelemente, Steuervorrichtungen, Vorrichtungen zur Laserlichtleitung, wie z.B. Glasfaserleitungen oder Hohlleiter, sowie ein Handstück, das ein Behandlungsinstrument bildet. Ferner können solche Laserbehandlungsgeräte Stromversorgungselemente und Anzeigen, wie z.B. Monitore oder Lampen umfassen, die üblicherweise in Gehäuseelementen angeordnet oder vorgesehen sind.

Medizinische Laserbehandlungsgeräte werden für unterschiedliche medizinische Zwecke und in unterschiedlichen Ausführungen eingesetzt. Je nach Einsatzbereich müssen die Laserbehandlungsgeräte unterschiedliche Spezifikationen aufweisen, insbesondere was die Charakteristik des von ihnen abgegebenem Laserlichts zur medizinischen Behandlung betrifft.

Für einen umfassenden Einsatz medizinischer Laserbehandlungsgeräte für unterschiedliche Anwendungen ist die behandelnde Person daher dazu gezwungen, unterschiedliche Laserbehandlungsgeräte einzusetzen, insbesondere zum einen ein Laserbehandlungsgerät, das ein cw-(Continuous Wave)-Laserlicht emittieren kann, zum anderen ein Laserbehandlungsgerät, das eine gepulstes Laserlicht emittieren kann. Die Beschaffung mehrerer Laserbehandlungsgeräte ist jedoch zum einen sehr kostenintensiv, zum anderen ist der teilweise erforderliche oder wünschenswerte Einsatz unterschiedlicher Laserbehandlungsgeräte während der Behandlung sehr umständlich und teilweise unter den gegebenen Bedingungen, insbesondere im gegebenen Zeitrahmen, nur schwer oder gar nicht möglich.

Aus der DE 198 44 719 A1 ist ferner eine Laserbehandlungsvorrichtung bekannt, die einen Festkörperlaser zur Erzeugung eines Laserstrahls und eine Anregungslichtquelle umfaßt, die den Festkörperlaser anregt, so daß dieser ein kontinuierliches Laserlicht, ein cw-Laserlicht, emittiert. Das in der DE 198 44 719 A1 offenbarte Gerät umfaßt ferner zwei unterschiedliche, voneinander unabhängige optische Systeme und ein System zur Umschaltung des optischen Pfades des Laserlichts, mittels dem das von einem Festkörperlaser erzeugte cw-Laserlicht entweder in das eine oder in das andere optische System gelenkt wird, wobei dieses Umschaltsystem durch einen Schwenkspiegel realisiert wird, der in den Strahlengang des von dem Festkörper erzeugten kontinuierlichen Laserstrahls hineingefahren wird, um das Laserlicht in ein erstes optisches System zu lenken bzw. aus dem Verlauf des Laserstrahls herausgefahren wird, um das Laserlicht unabgelenkt in ein zweites optisches System laufen zu lassen.

Ein solches System hat den Nachteil, daß es relativ komplex aufgebaut ist und insbesondere aufgrund der erforderlichen mechanischen Verstellung des Schwenkspiegels störanfällig ist. Ferner ist der Umschaltvorgang zeitaufwendig und es kann lediglich auf Laserlicht einer einzelnen Laserlichtquelle zugegriffen werden, so daß die Variations-und Einsatzmöglichkeiten eines solchen Systems beschränkt sind.

Es ist daher Aufgabe der vorliegenden Erfindung, ein medizinisches Laserbehandlungsgerät zur Verfügung zu stellen, das variabler einsetzbar ist, wobei unterschiedliche Laserlichtquellen nutzbar sind und ferner eine schnelle und störunanfällige Umschaltung zwischen einzelnen Betriebsmodi des Geräts verwirklicht werden kann.

Diese Aufgabe wird durch ein medizinisches Laserbehandlungsgerät gemäß Anspruch 1 gelöst, die Ansprüche 2 bis 18 betreffen besonders vorteilhafte Ausführungsformen des erfindungsgemäßen medizinischen Laserbehandlungsgeräts.

Gemäß der Erfindung umfaßt das Laserbehandlungsgerät, das zwischen einem cw-Betriebsmodus und einem Pulsbetriebsmodus schaltbar ausgebildet ist, mindestens eine erste Laserlichtquelle zur Erzeugung eines cw-Laserstrahls und eine zweite Laserlichtquelle zur Erzeugung eines gepulsten Laserstrahls, wobei die zweite Laserlichtquelle von mindestens einer ersten Laserlichtquelle gepumpt bzw. angeregt wird.

Erfindungsgemäß wird in dem cw-Betriebsmodus des Laserbehandlungsgeräts der cw-Laserstrahl mindestens einer der mindestens einen ersten Laserlichtquelle zur Behandlung oder Diagnose verwendet, während in dem Pulsbetriebsmodus der gepulste Laserstrahl der zweiten Laserlichtquelle zur Behandlung verwendet wird. Erfindungsgemäß ist ferner das Laserbehandlungsgerät so ausgelegt, daß die zweite Laserlichtquelle kontinuierlich von mindestens einer der mindestens einen ersten Laserlichtquelle gepumpt wird, auch wenn sich das Laserbehandlungsgerät in dem cw-Betriebsmodus befindet. Dies führt dazu, daß zumindest ein bestimmter Anteil der Laserlichtleistung der mindestens einen ersten Laserlichtquelle kontinuierlich dafür verwendet wird, die zweite Laserlichtquelle zu pumpen bzw. anzuregen, so daß dieser Anteil nicht für die Laserbehanldung an sich mit einem cw-Laserstrahl zur Verfügung steht, die zweite Laserlichtquelle sich jedoch ständig in angeregtem Zustand befindet.

Das erfindungegemäße Laserbehandlungsgerät hat insbesondere den Vorteil, daß zwei unterschiedliche Lasersysteme zur Erzeugung eines cw-Laserstrahls einerseits und eines gepulsten Laserstrahls andererseits in einem einzelnen Gerät einsetzbar sind, was die Bandbreite der möglichen Behandlung und die entsprechende Variabilität deutlich erhöht, insbesondere ist es aufgrund des Vorsehens einer zweiten Laserlichtquelle möglich, einen sehr hochenergetischen gepulsten Laserstrahl zur Verfügung zu stellen, der nicht realisierbar wäre, wenn lediglich ein cw-Laserlicht beispielsweise mittels eines Güteschalters oder ähnlichem in ein gepulstes Laserlicht umgewandelt wird.

Da erfindungsgemäß darüber hinaus die zweite Laserlichtquelle kontinuierlich angeregt bzw. gepumpt wird, sie sich daher in einem kontinuierlichen Betriebszustand befindet, ist ein Umschalten von einem cw-Betriebsmodus in einen Pulsbetriebsmodus ohne Zeitverzögerung möglich, da ein "Anfahren" der Pulslaserlichtquelle nicht erforderlich ist. Es soll an dieser Stelle angemerkt werden, daß das "Anfahren" einer Pulslaserlichtquelle je nach System in der Regel wenigstens 30 Sekunden benötigt und ggf. mehr als eine Minute vergehen kann, bis die Pulslaserlichtquelle stabil läuft, so daß eine behandelnde Person beim Umschalten von einem cw-Betriebsmodus in einen Pulsbetriebsmodus lange Wartezeiten hat, was insbesondere bei einem häufigen Wechsel der Betriebsarten, wie es insbesondere im Dentalbereich sehr oft wünschenswert ist, zu unakzeptablen Verzögerungen führen würde.

Bevorzugt ist es im cw-Betriebsmodus vorgesehen, daß im Vergleich zum Pulsbetriebsmodus nur ein geringerer Teil der Leistung der mindestens einen ersten Lichtquelle weiterhin als Pumpstrahlung für die zweite Laserlichtquelle verwendet wird, so daß der zweiten Laserlichtquelle zwar eine geringere Pumpleistung zur Verfügung stellt, diese Verringerung der Leistung aber so ausgewählt ist, daß der Verstärker nicht im Pulsbetrieb zusammenbricht und eine Rückkehr in den normalen Betrieb mit voller Pumpleistung sehr schnell erfolgen kann.

Bei einer anderen Ausführungsform ist es auch möglich, daß sowohl im cw-Betriebsmodus als auch im Pulsbetriebsmodus immer eine konstante Pumpleistung zur Verfügung gestellt wird. Dies kann zum einen dadurch geschehen, daß immer ein feststehender Anteil der von der mindestens einen ersten Lichtquelle erzeugten Leistung als Pumpstrahlung ausgekoppelt wird, zum anderen kann dies durch das Vorsehen mehrerer erster Lichtquellen, beispielsweise mehrerer Laserdioden, verwirklicht werden, wobei mindestens eine der ersten Laserlichtquellen, bevorzugt der Laserdioden, ausschließlich für das Pumpen der zweiten Laserlichtquelle verwendet wird, während weitere erste Laserlichtquellen zur Erzeugung des cw-Laserstrahls zur Behandlung dienen.

Bei einer Ausführungsform, bei der nur eine erste Laserlichtquelle verwendet wird bzw. mehrere erste Laserlichtquellen, beispielsweise Laserdioden, parallel verwendet werden und ein Teil der Laserleistung des erzeugten cw-Laserstrahls ausgekoppelt wird, eignet sich insbesondere als Auskoppelvorrichtung ein Strahlteiler, insbesondere ein Strahlteiler, der von der Polarisationsrichtung des einfallenden Lichts abhängig ist, wobei die Polarisation des auf den Strahlteiler auftreffenden Laserlichts, das von der mindestens einen ersten Laserlichtquelle erzeugt wird, bevorzugt mittels eines vorgeschalteten Elements zur Steuerung der Polarisationsrichtung regelbar ist. Bei einem solchen Element kann es sich beispielsweise um eine drehbare Halbwellenplatte handeln, die in den Strahlengang des von der mindestens einen ersten Laserlichtquelle erzeugten cw-Laserlichts gesetzt wird.

Als erste Laserlichtquellen eignen sich insbesondere Festkörperlaser, vor allem Laserdioden, während für die zweite Laserlichtquelle insbesondere ein Scheibenlaser geeignet ist. Ein Scheibenlaser hat den Vorteil, daß der für den Scheibenlaser eingesetzte Kristall sehr effektiv von nahezu allen Seiten gekühlt werden kann. Da der Durchmesser des Pumpstrahls in der Regel sehr viel größer ist als die Dicke der verwendeten dotierten Kristallscheibe, wird das Aufbauen einer sogenannten "thermischen Linse" stark unterdrückt, d.h. es wird vermieden, daß durch eine unterschiedliche Aufwärmung des Kristalls aufgrund der Tatsache, daß der Pumpstrahl eine unterschiedliche Intensitätsverteilung aufweist und in der Regel Gauß-verteilt ist, Veränderungen, insbesondere Verzerrungen des Kristalls auftreten, die zu ungewünschten Reflexionen führen können.

Ein Scheibenlaser ist daher auch im Vergleich zu anderen Lasersystemen weniger anfällig gegenüber Änderungen der Pumpleistung und bei gleichbleibend guter Strahlqualität in der Leistung skalierbar, was insbesondere bedeutsam für das erfindungsgemäße Laserbehandlungsgerät ist, da hier je nach Modus die zweite Laserlichtquelle mit unterschiedlicher Leistung gepumpt wird, wenn bespielsweise in einem cw-Betriebsmodus ein größerer Anteil der cw-Laserstrahlung der mindestens einen ersten Lichtquelle zur Behandlung ausgekoppelt wird und lediglich ein geringerer Teil dieser Leistung zum weiteren kontinuierlichen Pumpen bzw. Anregen der zweiten Laserlichtquelle, hier des Scheibenlasers, verwendet wird.

Je nach gewünschtem Behandlungsverfahren können dann das von der mindestens einen ersten Lichtquelle und/oder das von der zweiten Laserlichtquelle erzeugte cw-Laserlicht und/oder das gepulste Laserlicht abgeschirmt werden, beispielsweise durch Shutter, so daß entweder die Behandlung ausschließlich mit einem Pulslaserstrahl, ausschließlich mit einem cw-Laserstrahl oder auch parallel mit beiden Laserstrahlen durchgeführt wird. Es kann natürlich auch für eine Unterbrechung der Behandlung sämtliche Laserstrahlung abgeschirmt wird, ohne die Laserlichtquellen selbst auszuschalten.

Je nach Wahl des Betriebsmodus kann das medizinische Laserbehandlungsgerät daher für unterschiedliche medizinische Zwecke eingesetzt werden. Dies ist auf die Wechselwirkung des Laserlichts mit dem zu bearbeitenden Material, in der Regel biologisches Material, insbesondere Gewebe, zurückzuführen, die neben der Art des zu behandelnden Gewebes, der eingestrahlten Wellenlänge und -leistung auch von der gewählten Pulsdauer abhängt bzw. davon, ob gepulst oder mit einer kontinuierlichen Einstrahlung gemäß der Erfindung gearbeitet wird und welche Energien kontinuierlich oder pro Puls ausgesendet und übertragen werden.

Das medizinische Laserbehandlungsgerät gemäß der Erfindung stellt daher eine "Multi-Use"-Vorrichtung zur Verfügung, die für eine weite Bandbreite von Behandlungs- und Diagnoseverfahren eingesetzt werden kann, was zum einen wesentlich kostengünstiger ist als der Einsatz mehrerer Laserbehandlungsgeräte, zum anderen die Bedienbarkeit und den Komfort deutlich erhöht, da die zu behandelnde Person, in der Regel ein Arzt, bei einem Gerät durch einfaches Umschalten der Betriebsmodi ohne Wechsel der Geräte unterschiedliche Behandlungen bzw. Behandlungsschritte schnell und komfortabel durchführen kann.

Bei einer bevorzugten Ausführungsform umfaßt das erfindungsgemäße medizinische Laserbehandlungsgerät mindestens zwei Pulsbetriebsmodi, die unterschiedliche Laserpulsdauern und/oder unterschiedliche Pulsfolgefrequenzen des abgestrahlten Laserlichts aufweisen.

Im cw-Betriebsmodus des erfindungsgemäßen medizinischen Laserbehandlungsgeräts wird insbesondere eine thermische Wechselwirkung des abgestrahlten Laserlichts mit dem Gewebe erreicht. Das medizinische Laserbehandlungsgerät im cw-Betriebsmodus eignet sich daher insbesondere zur Koagulation und zur photodynamischen Therapie (PDT = Photodynamic-Therapy).

Bevorzugt ist das medizinische Laserbehandlungsgerät ferner mit einer Steuervorrichtung zur Steuerung der Laserlichtleistung versehen, wobei im cw-Betriebsmodus des Laserbehandlungsgeräts zur Koagulation bevorzugt Leistungswerte bis zu 3 Watt, bevorzugt etwa 2 Watt eingesetzt werden.

Der bzw. die Pulsbetriebsmodi des erfindungsgemäßen medizinischen Laserbehandlungsgeräts werden bevorzugt zur Ablation insbesondere von hartem Gewebe, wie organischem Hartgewebe, z.B. Zahnmaterial, insbesondere kariösem Zahnmaterial, eingesetzt. Bevorzugt weist einer der Betriebsmodi des medizinischen Laserbehandlungsgeräts Pulsdauern auf, die kürzer als 1 ps sind. Zur Ablation sind vor allem Pulsdauern im fs-Bereich bevorzugt, insbesondere in einem Bereich von 1 bis 1000 fs, bevorzugt in einem Bereich bis 700 fs und insbesondere von 5 bis 500 fs.

In diesen Betriebsmodi führt das erfindungsgemäße medizinische Laserbehandlungsgerät im wesentlichen zu einer nicht-thermischen Wechselwirkung mit dem zu behandelnden harten Gewebe, so daß die Ablation im wesentlichen ohne Erhitzung des umgebenden Gewebes, insbesondere des umgebenden harten Gewebes, durchgeführt wird.

Ein weiterer bevorzugter Betriebsmodus des erfindungsgemäßen medizinischen Laserbehandlungsgeräts weist Pulslängen im Pikosekundenbereich auf, insbesondere von 20 ps bis 500 ps. Eine bevorzugte Pulslänge für diesen Betriebsmodus liegt bei etwa 100 ps.

Dieser Betriebsmodus ist besonders für ein Versiegeln von Hartgewebeoberflächen (sealing) geeignet.

Bei einem weiteren bevorzugten Betriebsmodus des erfmdungsgemäßen medizinischen Laserbehandlungsgeräts werden Laserpulse mit einer Pulsdauer von einer Nanosekunde oder länger erzeugt. Ähnlich wie bei dem cw-Betriebsmodus eignen sich diese sehr langen Pulsdauern insbesondere zur Koagulation von Gewebe.

Eine Kombination mindestens zweier der oben genannten Betriebsmodi bzw. weiterer Betriebsmodi ermöglicht daher den vielfältigen Einsatz des medizinischen Laserbehandlungsgeräts. Die behandelnde Person, insbesondere der Arzt, ist daher in der Lage, durch einfaches Umschalten des Betriebsmodus ohne Wechseln des Gerätes und ohne Zeitverzögerung andere medizinische Effekte zu erzielen. So ist es beispielsweise für einen Zahnarzt möglich, während der Ablation von Zahnmaterial von einem Pulsbetriebsmodus auf einen cw-Betriebsmodus umzuschalten, wenn eine Blutung auftritt, so daß umgehend die Blutung mittels Koagulation gestillt werden kann. Danach kann umgehend wieder zur Ablation übergegangen werden, ohne daß der Zahnarzt die Aufmerksamkeit von seinem Patienten abwenden muß, um das Behandlungsgerät zu wechseln, wobei bei dem Umschalten in den Pulsbetriebsmodus insbesondere die durch das erfindungsgemäße Laserbehandlungssystem ermöglichte nahezu verzögerungsfreie Umschaltung von hoher Bedeutung ist. Der Patient kann daher schneller, schonender, besser und kostengünstiger behandelt werden.

Bevorzugt weist das medizinische Laserbehandlungsgerät eine Laserlichtquelle auf, die ein Laserlicht abstrahlt, das eine Wellenlänge von λ = 750 nm bis λ = 1.100 nm aufweist. Diese Wellenlänge ist insbesondere deshalb bevorzugt, da die Absorption biologischen Gewebes in diesem Wellenlängenbereich relativ gering ist, so daß insbesondere in den Betriebsmodi, in denen eine thermische Wechselwirkung mit dem Gewebe zum Tragen kommt, eine hohe Eindringtiefe und dadurch eine gleichmäßige und wirksame Behandlung ermöglicht wird.

Dieser Bereich eignet sich daher insbesondere für die Gewebekoagulation, während für die Ablation eine hohe Absorption vorteilhaft ist. Eine hohe Absorption in Dentin wird insbesondere bei Kurzpulslasern aufgrund der hohen Intensität auch bei einer Wellenlänge von 780 nm erreicht. Eine höhere Absorption auch bei geringeren Intensitäten wird ferner insbesondere im UV-Bereich und bei Wellenlängen <500 nm, insbesondere bei 390 nm oder bei 248 nm (ArF-Laser) erreicht.

Als zweite Laserlichtquelle wird bevorzugt ein Yb:KGW-Laser, ein Yb:KYW-Laser oder ein Yb:YAG-Laser mit einer Wellenlängenabstrahlung von 1.030 nm eingesetzt. Ferner ist es auch möglich, einen Nd:YAG-Laser mit einer Wellenlängenabstrahlung von 1.064 nm oder einen Nd:YLF-Laser mit einer Wellenlängenabstrahlung von 1.053 nm einzusetzen. Ferner eignen sich als erste Laserlichtquelle insbesondere Dioden-Laser, die in einem Wellenlängenbereich von 805 nm bis 980 nm arbeiten, beispielsweise mit Emissionswellenlänge von 809 nm, 940 nm oder 980 nm.

Typische Parameter vorteilhafter Laser sind in der folgenden Tabelle angegeben:

| | | | | |
|---|---|---|---|---|
| Laser | Nd:YAG | Er:YAG | Diodenlaser | Yb:KGW |
| Leistung | LOW - 5kW | <200W | 1W->100W | <100W |
| Wellenlänge | 1064 nm | 2940 nm | 808, 1500 nm | Ca. 1030 um |
| Pulsdauer | Ca. 10 ns- cw | Ca. 10 ns - ms | cw | <1ps |

Bei einer weiterenen Ausführungsform umfaßt das medizinische Laserbehandlungsgerät ferner eine Scan-Vorrichtung zur Erzeugung eines Abtastmusters auf der zu behandelnden Fläche oder in dem zu behandelnden Bereich. Die Scan-Vorrichtung umfaßt bevorzugt steuerbare Spiegel, die den erzeugten Laserstrahl so ablenken, daß er eine vorgegebene, steuerbare x-y-Ablenkung bevorzugt periodisch durchläuft.

Die Scan-Vorrichtung ist bevorzugt durch einen Computer steuerbar, so daß das Abtastmuster, die Abtastfläche und die Abtastgeschwindigkeit den gewünschten Behandlungszielen angepaßt werden kann. Die Abtastgeschwindigkeit, die in der Einheit Strecke pro Zeit angegeben wird, liegt bevorzugt in Bereichen von 10 bis 1000 mm/s, insbesondere bei 100 bis 500 mm/s, wobei ein besonders bevorzugter Wert bei etwa 200 mm/s liegt. Die Abtastfrequenz, worunter der vollständige Durchlauf des Abtastmusters pro Zeit zu verstehen ist, hängt von der Abtastgeschwindigkeit einerseits und dem Abtastmuster und der Abtastfläche andererseits ab und liegt bevorzugt in einem Bereich von 1 Hz bis 1kHz, insbesondere bei 1 bis 100 Hz, wobei ein besonders bevorzugter Wert etwa 10 Hz beträgt.

Der Behandlungserfolg hängt neben der gewählten Wellenlänge und der Pulsdauer auch von der Pulsfolgefrequenz ab, die bevorzugt je nach Behandlungsmodus ebenfalls einstellbar ist.

Es hat sich herausgestellt, daß eine Ablation insbesondere bei einer kurzen Laserpulsdauer und einer hohen Pulsfolgefrequenz gefördert wird und besonders effektiv ist, während ein Versiegeln von harten Gewebeflächen (sealing) bei mittleren Pulslängen im Bereich von 20 ps bis 500 ps und niedrigeren Pulsfolgefrequenzen, insbesondere mit Pulsfolgefrequenzen, die kleiner sind als 1 kHz, besonders effektiv durchgeführt werden kann. Koagulation wird am besten bei sehr langen Pulsdauern oder bevorzugt mit einer cw-Strahlung erzeugt.

Bei einer bevorzugten Ausführungsform ist daher eine Steuervorrichtung vorgesehen, die in Kombination mit dem Betriebsmodus des Laserbehandlungsgeräts auch weitere charakteristische Abstrahl- bzw. Behandlungscharakteristika des Laserbehandlungsgeräts, insbesondere die Abtastgeschwindigkeit bzw. Abtastfrequenz und die Pulsfolgefrequenz steuert. Diese können automatisch vorgegeben sein oder auch manuell regelbar sein.

Bevorzugt ist daher das medizinische Laserbehandlungsgerät in einem Ablationsmodus betreibbar, bei dem sowohl eine kurze Pulsdauer t < 1 ps und eine hohe Pulsfolgefrequenz in einem Bereich von 1 kHz bis 50 kHz, insbesondere ca. 30 kHz realisiert sind.

In einem weiteren Modus, dem Versiegelungs- oder "Sealing"-Modus, sind das medizinische Laserbehandlungsgerät und/oder die Steuervorrichtung bevorzugt so ausgelegt, daß das medizinische Laserbehandlungsgerät Pulse mit einer Pulsdauer von 20 ps bis etwa 500 ps bei einer Pulsfolgefrequenz zur Behandlung erzeugt, die kleiner als 1 kHz ist.

In einem weiteren, "Koagulations-Modus" sind das medizinische Laserbehandlungsgerät und/oder die Steuervorrichtung bevorzugt so ausgelegt, daß das medizinische Laserbehandlungsgerät Laserlicht in einem cw-Modus zur Behandlung ausstrahlt, wobei die Scanner-Vorrichtung ausgeschaltet ist, so daß in dem Laserbehandlungsgerät ein im wesentlichen unbewegter oder orts-fester Laserstrahl erzeugt wird.

Bei einer bevorzugten Ausführungsform des medizinischen Laserbehandlungsgeräts ist ferner eine Schaltvorrichtung vorgesehen, die entweder eine Schaltung zwischen den oben genannten Kombinationsmodi, die sowohl die Betriebsmodi (insbesondere Pulsdauer bzw. cw-Modus) als auch die Abtastfrequenz des Scanners und die Pulsfolgefrequenz vorgeben, ermöglichen, oder aber eine getrennte Schaltung zwischen den Betriebsmodi und anderer charakteristischer Arbeitsdaten des medizinischen Laserbehandlungsgeräts ermöglichen. Insbesondere umfaßt die Schaltvorrichtung auch Elemente zur bevorzugt stufenlosen Leistungsregelung der medizinischen Laserbehandlungsvorrichtung.

Bei einer weiteren bevorzugten Ausführungsform des medizinischen Laserbehandlungsgeräts ist ferner ein sichtbarer Hilfs-Laserstrahl vorgesehen, der sowohl vor als auch während der Behandlung erzeugt werden kann, um der zu behandelnden Person insbesondere bei einer "Non-Contact"-Behandlung die Behandlung zu erleichtern. Die erfindungsgemäße medizinische Laserbehandlungsvorrichtung kann dazu beispielsweise eine Vorrichtung zur Frequenzverdoppelung aufweisen, wobei bevorzugt ein Teil des von der mindestens einen ersten Laserlichtquelle erzeugten cw-Laserstrahls ausgekoppelt und als Hilfs-Laserstrahl verwendet wird. Es ist jedoch auch möglich, eine zusätzliche Laserlichtquelle, insbesondere eine Laserdiode, einzusetzen, die im sichtbaren Wellenlängenbereich abstrahlt. Besonders bevorzugt ist es, daß diese Hilfs-Laserlichtquelle bzw. die Vorrichtungen zur Erzeugung eines Hilfs-Laserstrahls den Betriebszustand der medizinischen Laserbehandlungsvorrichtung anzeigt.

Dies kann z.B. dadurch geschehen, daß das Laserlicht des Hilfs-Laserlichtstrahls blinkt, wenn kein Behandlungsstrahl erzeugt wird, und kontinuierlich strahlt, wenn ein Behandlungslaserlicht ausgestrahlt wird, sei es kontinuierlich oder gepulst. Selbstverständlich ist es auch möglich, daß die Wellenlänge des abgestrahlten Hilfs-Laserlichts je nach Betriebszustand des Laserbehandlungsgeräts verändert wird. Auch akustische Anzeigevorrichtungen zur Anzeige des Betriebszustandes können vorgesehen werden, entweder isoliert oder auch in Kombination mit den oben genannten Vorrichtungen.

Diese und weitere Vorteile und Merkmale des erfindungsgemäßen medizinischen Laserbehandlungsgeräts werden anhand der nachfolgenden Figuren deutlich. Die schematischen Darstellungen zeigen:
- Fig. 1: in einer Übersichtsdarstellung Elemente einer Ausführungsform eines medizinischen Laserbehandlungsgeräts;
- Fig. 2: eine Darstellung einer Vorrichtung zur Erzeugung eines Hilfs-Laserstrahls gemäß einer Ausführungsform eines erfindungsgemäßen medizinischen Laserbehandlungsgeräts;
- Fig. 3: einen Aufbau einiger Elemente einer Ausführungsform eines medizinischen Laserbehandlungsgeräts;
- Fig. 4: Parameter für bevorzugte Betriebszustände eines erfindungsgemäßen medizinischen Laserbehandlungsgeräts;
- Fig. 5: einen Aufbau eines Stretchers, wie er in einer Ausführungsform des erfindungsgemäßen Laserbehandlungsgeräts eingesetzt werden kann;
- Fig. 6: einen Aufbau eines Kompressors, wie er in einer Ausführungsform des erfindungsgemäßen Laserbehandlungsgeräts eingesetzt werden kann; und
- Fig. 7.: schematisch einen Querschnitt durch einige Elemente eines Scheibenlasersystems.

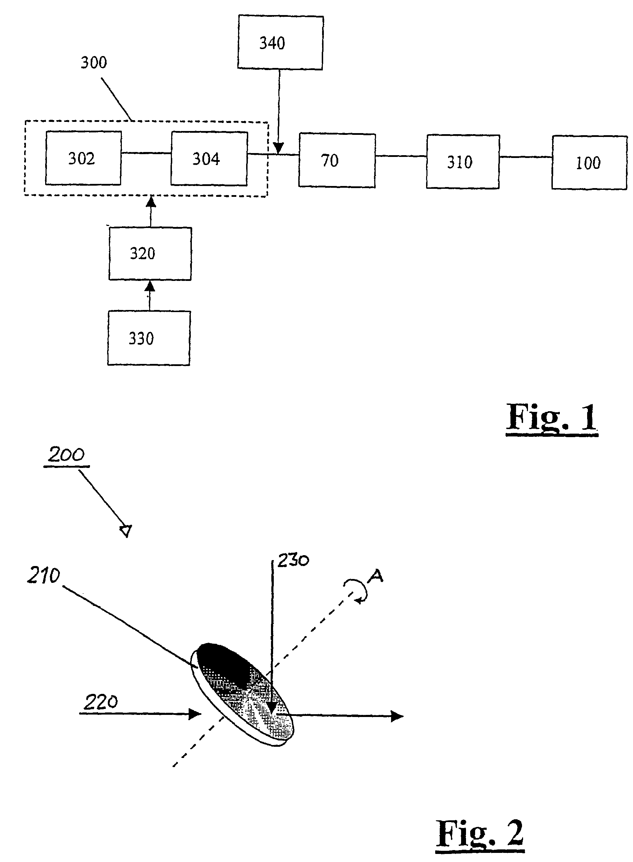

Fig. 1 zeigt schematisch in einer Übersichtsdarstellung einige Elemente einer Ausführungsform eines medizinischen Laserbehandlungsgeräts: Das medizinische Laserbehandlungsgerät dieser Ausführungsform umfaßt Laserlichtquellen 302, die eine erste Laserlichtquelle bestehend aus mehreren Laserdioden und einen Scheibenlaser als zweite Laserlichtquelle umfassen, und eine Vorrichtung 304 zur Steuerung der Betriebsmodi, die auch in einer kombinierten Lasereinheit 300 zusammengefaßt sein können.

Die Lasereinheit 300 bzw. die Laserlichtquellen 302 und die Vorrichtung 304 zur Steuerung der Betriebsmodi werden über eine Steuervorrichtung 320 geregelt, die wiederum durch eine Schaltvorrichtung 330 bedient werden kann, die von der zu bedienenden Person, in der Regel einem Arzt oder einem technischen Personal, betätigbar ist.

Ferner umfaßt das in Fig. 1 schematisch gezeigte medizinische Laserbehandlungsgerät eine Vorrichtung 340 zum Einkoppeln eines Hilfs-Laserstrahls, wobei darauf hingewiesen werden soll, daß diese Vorrichtung lediglich optional ist.

Der Behandlungslaserstrahl und gegebenenfalls der Hilfs-Laserstrahl werden über optische Elemente weitergeleitet, wobei hier lediglich symbolisch eine optische Übertragungsvorrichtung 70 dargestellt ist.

Ferner umfaßt die hier dargestellte Ausführungsform des medizinischen Laserbehandlungsgeräts auch eine steuerbare Scan-Vorrichtung 310, so daß auf den Laserstrahl eine Abtastbewegung übertragen werden kann.

Das gegebenenfalls mit einer Scanbewegung beaufschlagte Laserlicht wird an ein Handstück 100 weitergeleitet, das zur Handhabung durch die zu behandelnde Person dient. An dieser Stelle soll auch darauf hingewiesen werden, daß die Scan-Vorrichtung 310 durchaus auch in das Handstück 100 integriert sein kann.

Der in Fig. 1 dargestellte Aufbau soll lediglich die schematische Struktur verdeutlichen, selbstverständlich können die gezeigten Elemente in ihrer Reihenfolge ausgetauscht werden, einzelne Elemente weggelassen oder zusätzliche Elemente hinzugefügt werden, ohne vom Umfang der durch die Ansprüche beschriebenen Erfindung abzuweichen.

Fig. 2 zeigt schematisch eine Vorrichtung 200 zum Einkoppeln eines Hilfs-Laserstrahls, wie sie auch bei der in Fig. 1 gezeigten Ausführungsform einsetzbar ist (siehe bei 340 in Fig. 1). Ein Filter 210 wird dabei als dichromatischer Strahlteiler eingesetzt, der für ein Behandlungslaserlicht 220 im wesentlichen durchsichtig ist. Der Hilfs-Laserstrahl 230, der beispielsweise von einer Laserdiode erzeugt werden kann und im sichtbaren Wellenlängenbereich liegt, wird von dem Filter 210 reflektiert, so daß er mit dem Behandlungslaserstrahl 220 überlagert werden kann. Die Reflexion des Hilfs-Laserstrahls 230 wird bei der in Fig. 2 dargestellten Ausführungsform durch eine Reflexionsbeschichtung des Filters 210 sichergestellt.

Die Intensität des Behandlungs-Laserstrahls 220 kann durch eine Drehung des Filters 210 um seine Achse, wie durch den Pfeil A angedeutet, stufenlos verändert werden. Die Intensität des Hilfs-Laserstrahls wird durch die Drehung des Filters 210 im wesentlichen nicht beeinflußt.

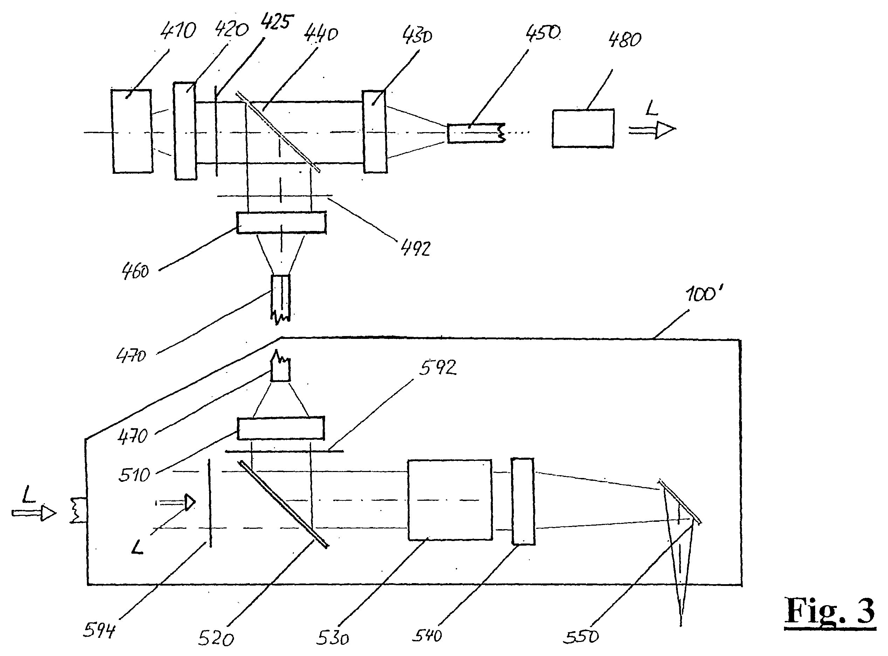

Fig. 3 zeigt schematisch Elemente einer weiteren Ausführungsform eines erfindungsgemäßen medizinischen Laserbehandlungsgeräts.

Die im oberen Teil der Fig. 3 gezeigte Laserlichtquelle umfaßt eine Laserdiode 410 als erste Laserlichtquelle und einen Scheibenlaser 480 als zweite Laserlichtquelle. Das von der Laserdiode 410 emittierte kontinuierliche cw-Laserlicht wird über einen Kollimator 420 auf einen Strahlteiler 440 gerichtet. Der Strahlteiler 440 koppelt einen Teil der cw-Laserleistung aus, der über eine Fokussierlinse 460 und eine Lichtleitfaser 470 zu einem Handstück 100' weitergeleitet wird.

Der nicht von dem Strahlteiler 440 ausgekoppelte Teil des von der Laserdiode 410 emittierten cw-Lichts wird kontinuierlich über eine Fokussierlinse 430 und eine Lichtleitfaser 450 zu dem Scheibenlaser 480 weitergeleitet, der ein gepulstes Laserlicht L emittiert.

Dem schematisch dargestellten Handstück 100' werden sowohl das von dem Strahlteiler 440 ausgekoppelte cw-Licht der Laserdiode 410 als auch das von dem Scheibenlaser 480 emittierte gepulste Laserlicht L zugeführt.

Das von dem Strahlteiler 440 ausgekoppelte und über die Lichtleitfaser 470 dem Handstück 100' zugeführte cw-Laserlicht der Laserdiode 410 wird von einem Einkoppelspiegel 520 durch eine Scannervorrichtung 530 über eine Fokussierlinse 540 und einen Umlenkspiegel 550 so weitergeleitet und aus dem Handstück 100' herausgeleitet, daß es zur Behandlung oder Diagnose verwendet werden kann.

Das von dem Scheibenlaser 480 emittierte gepulste Laserlicht L wird ebenfalls in das Handstück 100' geführt und durchläuft den für diese Wellenlänge durchsichtigen Einkoppelspiegel 520 und danach, analog zu dem vorher beschriebenen, von dem Strahlteiler ausgekoppelten cw-Laserlicht der Laserdiode 410, den Scanner 530, die Fokussierlinse 540 und wird über einen Umlenkspiegel 550 aus dem Handstück 100' nach außen zur Behandlung oder Diagnose weitergeleitet.

Die in Fig. 3 gezeigte Ausführungsform des erfindungsgemäßen medizinischen Laserbehandlungsgerätes kann mehrere Vorrichtungen umfassen, mit denen der Benutzer auswählen kann, welches emittierte Licht er für die Behandlung oder die Diagnose verwenden will. In Fig. 3 sind beispielhaft Shutter 492, 592, 594 vorgesehen, die von dem Benutzer steuerbar sind, so daß mit diesen das cw- und/oder das gepulste Laserlicht abgeschirmt oder durchgelassen werden können.

Bei der hier gezeigten Ausführungsform ist ein Shutter 492 zur möglichen Abschirmung eines cw-Laserlichts bereits außerhalb des Handstücks 100' vorgesehen, während zwei weitere Shutter 592, 594 in dem Handstück 100' vorgesehen sind. Selbstverständlich ist es auch möglich, die Shutter 492, 592, 594 anders zu positionieren, beispielsweise auf den Shutter 492 oder 592 zu verzichten oder anstelle der Shutter andere Elemente mit vergleichbarer Wirkung, beispielsweise Spiegelvorrichtung, einzusetzen.

Der Benutzer kann es daher steuern, ob er lediglich das vom Strahlteiler 440 ausgekoppelte cw-Licht der Laserdiode 410 oder das vom Scheibenlaser 480 emittierte gepulste Laserlicht L oder aber eine Kombination beider emittierter Strahlungen nutzen will. Selbstverständlich ist es auch möglich, mittels der Shutter schnell und unproblematisch eine Abstrahlung von Laserlicht aus dem Handstück 100' vollständig zu unterbinden, ohne die Laserlichtquellen an sich abzuschalten.

Bei der gezeigten Ausführungsform ist ein Diodenlaser 410 eingesetzt, der mit einer Wellenlänge von λ = 809 nm emittiert und besonders gut zur Koagulation geeignet ist, während der Scheibenlaser 480 mit einer Wellenlänge von λ = 1030 nm emittiert. Anstelle des Scheibenlasers könnte auch ein üblicher Yb:KGW-Lasers, ein Yb:KYW-Laser oder auch ein Nd:YAG-Laser eingesetzt werden, der Licht in einer Wellenlänge von λ = 1064 nm emittiert. Auch können anstelle der Laserdiode 410 andere cw-Laserquellen eingesetzt werden.

Der Nutzer hat daher die Möglichkeit, zwischen cw-Laserstrahlung und Pumplaserstrahlung auszuwählen, er hat auch die Möglichkeit, gleichzeitig cw-Laserlicht und gepulstes Laserlicht überlagert einzusetzen.

Zu der in Fig. 3 gezeigten Ausführungsform soll noch angemerkt werden, daß der Strahlteiler 440 kontinuierlich einen Teil der von der Laserdiode 410 emittierten Laserlicht an den Scheibenlaser 480 weitergibt, so daß keine oder nur für den Betrieb des Scheibenlasers 480 unschädliche Energiefluktuationen in dem nachfolgenden regenerativen Verstärker bzw. in dem Scheibenlaser 480 auftreten. Grundsätzlich ist es möglich, den Strahlteiler 440 so auszubilden, daß ein Teil des von der Laserdiode 410 erzeugten Laserlichts nur dann zur Behandlung ausgekoppelt wird, wenn das von diesem erzeugte Licht auch tatsächlich zur Behandlung und Diagnose verwendet wird. Beispielsweise kann der Strahlteiler 440 beweglich angeordnet sein und weggeklappt werden, wenn sämtliche Pumpleistungen der Laserdiode 410 für das nachfolgende Lasersystem 480 bereitgestellt werden sollen.

Bevorzugt werden anstelle einer Laserdiode 410 auch mehrere Laserdioden eingesetzt. Neben der in Figur 3 gezeigten Ausführungsform ist es auch möglich, zusätzlich eine weitere Laserdiode (oder eine sonstige Laserstrahlquelle) als weitere erste Laserlichtquelle unabhängig von der Laserdiode 410 anzuordnen und an einer beliebigen Stelle in das Laserbehandlungsgerät, bevorzugt in das Handstück 100' einzukoppeln, während die Laserdiode 410 als eine erste Laserlichtquelle hauptsächlich als Pumplaser für den Scheibenlaser 480 (oder eine andere zweite Laserlichtquelle) dient. Die Weiterleitung und die Einkopplung dieses (zusätzlichen) Laserlichts in das Handstück 100' wird auf Grund der Verwendung beispielsweise von lichtleitenden Fasern auf einfache Weise ermöglicht.

Eine solche Ausführungsform hat den Vorteil, daß es nicht unbedingt erforderlich ist, den in Figur 3 gezeigten Strahlteiler 440 vorzusehen, dennoch kann dieser weiterhin vorgesehen sein, um eine hohe Variabilität des Gesamtsystems sicherzustellen.

Die in Fig. 3 gezeigte Ausführungsform umfaßt ferner eine drehbare Halbwellenplatte 425, mit der die Polarisationsrichtung des Laserlichts beim Durchlauf durch diese Halbwellenplatte 425 beeinflußt werden kann. In diesem Falle handelt es sich bei dem Strahlteiler 440 um einen Strahlteiler, dessen Transmissions- bzw. Reflexionskoeffizient von der Polarisationsrichtung des auf ihn auftreffenden Laserlichts abhängig ist, so daß durch eine Drehung der drehbaren Halbwellenplatte 425 der Anteil des cw-Laserlichts bestimmt werden kann, der durch den Strahlteiler 440 ausgekoppelt wird bzw. der an den Scheibenlaser 480 weitergegeben wird. Dadurch wird auf einfache Weise eine stufenlose Steuerung der Leistungen des cw-Laserlichts ermöglicht, die zum einen kontinuierlich an den Scheibenlaser 480 (oder eine andere zweite Laserlichtvorrichtung) weitergegeben werden, so daß der Pulsbetrieb aufrecht erhalten werden kann, zum anderen ausgekoppelt und in das Handstück 100' eingekoppelt wird. Es soll an dieser Stelle darauf hingewiesen werden, daß die drehbare Halbwellenplatte 425 und der Strahlteiler 440 so ausgerichtet bzw. ausgebildet sein könnten, daß nahezu 100% der von der Laserdiode 410 emittierten cw-Strahlung an den Scheibenlaser 480 weitergegeben werden kann, wobei andererseits auch eine deutliche Reduzierung der "Pumpleistung" an den Scheibenlaser 480 möglich ist.

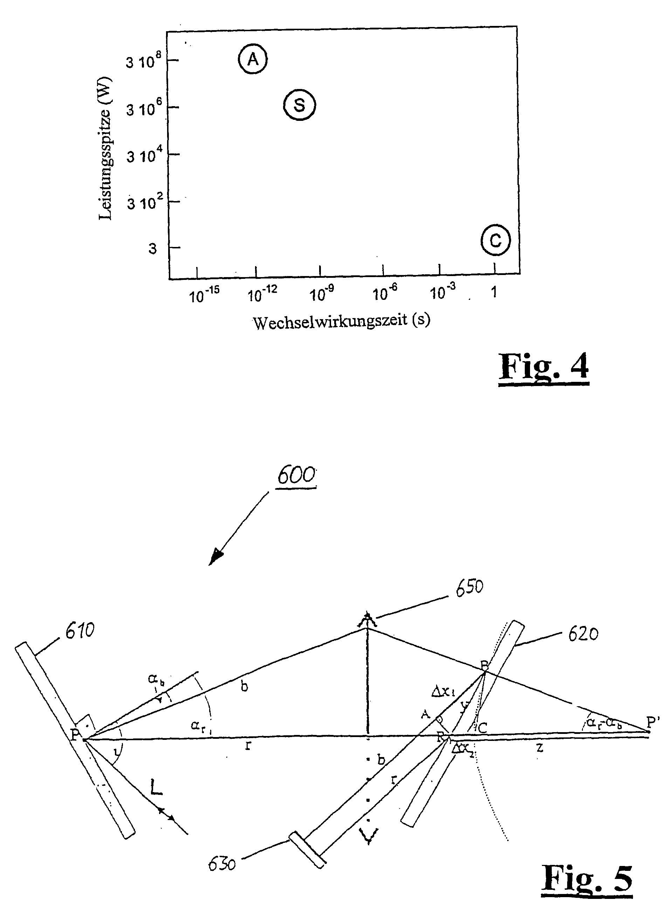

Fig. 4 zeigt bevorzugte Parameter für unterschiedliche Betriebszustände des erfindungsgemäßen medizinischen Laserbehandlungsgeräts, wobei sowohl die Wechselwirkungszeit in Sekunden als auch die Leistungsspitze in dem gewählten Betriebsmodus angegeben ist.

Bei dem mit "C" gekennzeichneten Bereich handelt es sich um einen cw-Betriebsmodus mit einer kontinuierlichen Leistung von etwa 3 Watt. Die Wechselwirkungszeit ist hier als eine Sekunde angegeben, wobei dies nicht der maximalen Behandlungsdauer entsprechen muß, vielmehr in der Regel eine mehrere Sekunden dauernde Behandlung durchgeführt wird.

Die mit "S" und "A" gekennzeichneten Bereiche entsprechen jeweils Pulsbetriebsmodi des erfindungsgemäßen medizinischen Laserbehandlungsgeräts, wobei bei dem mit S gekennzeichneten Bereich eine Pulsenergie von 300 µJ bei einer Pulsdauer von ungefähr 100 ps realisiert wird, was einer maximalen Pulsleistung von etwa 3 x 10⁶ W entspricht. Dieser Bereich eignet sich insbesondere zum Versiegeln von Hartsubstanz, insbesondere im zahnärztlichen Bereich.

Bei dem mit "A" gekennzeichneten Bereich wird ebenfalls eine Pulsenergie von 300 µJ, allerdings während einer Pulsdauer von unter 1 ps abgegeben, was einer Pulsspitzenleistung von etwa 3 x 10⁸ W entspricht. Dieser Modus eignet sich insbesondere zur Ablation von Hart-Material, insbesondere bei der zahnärztlichen Behandlung.

Selbstverständlich können auch weitere Bereiche und Betriebsmodi eingesetzt werden. Die in Fig. 4 dargestellten Bereiche zeigen lediglich besonders bevorzugte Betriebsbereiche einer Ausführungsform eines medizinischen Laserbehandlungsgeräts.

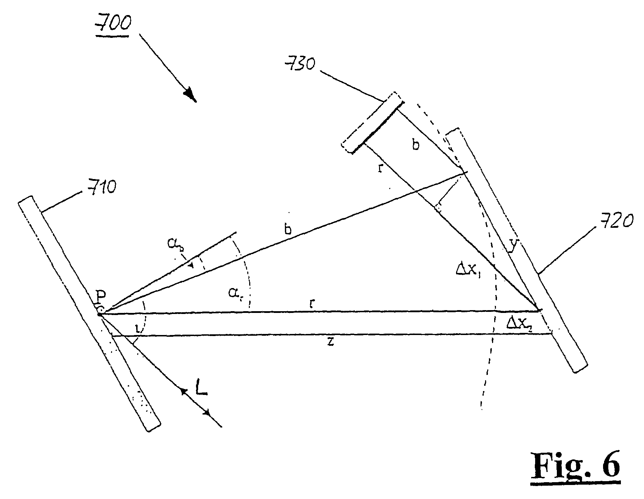

In den Fig. 5 und 6 sind Elemente zur Pulslängenveränderung dargestellt, wie sie z.B. im Zusammenhang mit dem CPA-Verfahren (Chirped Pulse Amplification) eingesetzt werden.

In Fig. 5 ist dabei ein "Stretcher" 600 gezeigt, der einen eintretenden Laserpuls zeitlich verlängert, während in Fig. 6 ein "Kompressor" 700 dargestellt ist, der einen eintretenden Laserpuls verkürzt bzw. komprimiert.

Bei den gezeigten Elementen werden die dispersiven Eigenschaften von Gittern und Prismen verwendet, um die eintreffenden Pulse auseinanderzuziehen oder zu komprimieren.

Tritt, wie in Fig. 5 gezeigt, ein Laserpuls L unter dem Winkel τ im Punkt P auf ein Reflexionsgitter 610, so werden die spektralen Anteile des Pulses L unter verschiedenen Winkeln reflektiert. Die Strahlen b und r, die die unterschiedlichen Wellenlängen repräsentieren, verlassen das Gitter 610 in erster Ordnung unter den Winkeln α₃ und αᵣ. Eine Linse 650 ist ferner so positioniert, daß der Punkt P 1:1 in Punkt P' abgebildet würde.

Zusätzlich zur Linse 650 ist ein zweites Gitter 620 so positioniert, daß alle Strahlen, die unter einem bestimmten Winkel an dem Gitter 610 reflektiert werden, unter diesem Winkel auf das Gitter 620 treffen. Da daher der Beugungswinkel an Gitter 620 für alle Strahlen genau τ ist, verlaufen alle spektralen Anteile nach dem Gitter 620 wieder parallel. Das Gitter 620 ist längs der mit Z gekennzeichneten Strecke verschiebbar, daher ergeben sich nach dem zweiten Gitter einstellbare, unterschiedliche Weglängen für die Strahlen r und b.

Die verschiedenen spektralen Anteile erfahren daher unterschiedliche Laufzeiten, so daß sich nach einer Reflexion in sich selbst eine Verlängerung des Pulses ergibt ("Chirp").

Während im Stretcher 600 ein positiver Chirp erzeugt wird, ist der Chirp in dem in Fig. 6 gezeigten Kompressor 700 negativ. Der in Fig. 6 gezeigte Kompressor umfaßt, analog zu dem in Fig. 5 gezeigten Stretcher, ein erstes Gitter 710, ein zweites Gitter 720 und einen Spiegel 730.

Durch ein Verschieben des zweiten Gitters 620 in dem Stretcher 600 bzw. ein Verschieben des zweiten Gitters 720 in dem Kompressor 700 können daher die Pulsdauern des gepulsten Laserlichts L in weiten Bereichen verändert werden.

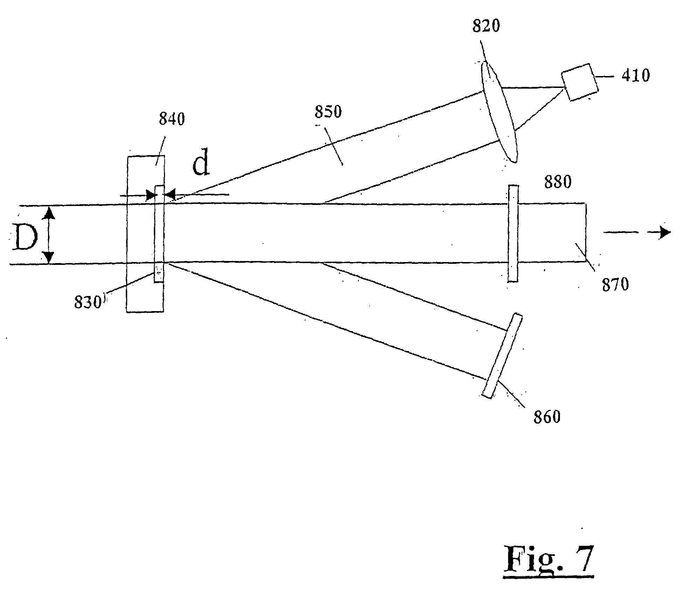

Fig. 7 zeigt schematisch einen Querschnitt durch einige Bauelemente eines Scheibenlasersystems, wie es in dem erfindungsgemäßen Laserbehandlungsgerät als zweite Laserlichtquelle eingesetzt werden kann, wobei in Fig. 7 zusätzlich auch Elemente des ersten Laserlichtsystems schematsich dargestellt sind, hier insbesondere die Laserdiode 410, die als erste Laserlichtquelle verwendet wird. In Fig. 7 sind jedoch lediglich die Elemente gezeigt, die für das Pumpen bzw. Anregen des Scheibenlasers erforderlich sind, während Elemente, die für ein teilweises Auskoppeln der von der Laserdiode 410 erzeugten Laserleistung erforderlich sind, in Fig. 7 zur Vereinfachung der Darstellung nicht gezeigt sind. Darüber hinaus ist auch lediglich ein Reflexionsweg gezeigt, um die Darstellung übersichtlich zu gestalten.

Das von der Laserdiode 410 erzeugte cw-Laserlicht durchläuft eine Linse 820 und trifft dann auf eine Kristallscheibe 830 des Scheibenlasers, die von einer Kühlvorrichtung 840 des Scheibenlasers von drei Seiten gekühlt wird.

Wie gut in Fig. 7 ersichtlich ist, ist der Durchmesser D des von der Laserdiode 410 erzeugten Pumplaserstrahls 850 sehr viel größer als die Dicke d der Kristallscheibe 830 des Scheibenlasers, wodurch eine extrem effektive Kühlung ermöglicht wird. Der Wärmefluß kann aufgrund der Dimensionierung des Durchmessers D des Pumplaserstrahls 850 und der Dicke d der Kristallscheibe 830 im wesentlichen als eindimensional angesehen werden, wodurch das Aufbauen einer thermischen Linse stark unterdrückt wird, was zu den oben beschriebenen Vorteilen führt.

Ferner ist in Fig. 7 schematisch gezeigt, daß der Pumplaserstrahl 850 mittels eines Reflexionselements 860, hier ein voll relektierender Spiegel, reflektiert wird. Über einen Auskoppelspiegel 880 wird ein Laserstrahl 870 ausgekoppelt, wobei es sich bei dem ausgekoppelten Laserstrahl 870 um einen gepulsten Laserstrahl handelt, der direkt für die Behandlung bzw. Diagnose eingesetzt werden kann.

Bei dem in Fig. 7 gezeigten Scheibenlasersystem handelt es sich um einen Yb:KGW-Scheibenlaser, der mit einer Wellenlänge von 1.026 nm emittiert und eine Emissionsbandbreite von ungeführt 15 nm aufweist. Es sind jedoch auch andere Materialien einsetzbar, insbesondere beispielsweise ein Yb:KYW-Scheibenlaser, auch Yb:YAG-Scheibenlaser oder Nd-dotierte Materialien können verwendet werden, wie z.B. ein Nd:glass-Scheibenlaser mit einer Emissionswellenlänge von etwa 1.054 nm und einer Emissionsbandbreite von circa 20 nm können verwendet werden.

### Bezugszeichenliste

- 70: optische Übertragungsvorrichtung
- 100, 100': Handstück
- 200: Vorrichtung zur Einkopplung eines Hilfs-Laserstrahls
- 210: Filter
- 220: Behandlungslaserlicht
- 230: Hilfs-Laserstrahl
- 300: Lasereinheit (mit erster und zweiter Laserlichtquelle)
- 302: Laserlichtquellen
- 304: Vorrichtung zur Steuerung der Betriebsmodi
- 320: Steuervorrichtung
- 330: Schaltvorrichtung
- 340: Vorrichtung zum Einkoppeln eines Hilfs-Laserstrahls
- 410: Laserdiode (erste Laserlichtquelle)
- 420: Kollimator
- 425: drehbare Halbwellenplatte
- 430: Fokussierlinse
- 440: Strahlleiter
- 450: Lichtleitfaser
- 460: Fokussierlinse
- 470: Lichtleitfaser
- 480: Scheibenlaser (zweite Laserlichtquelle)
- 492: Shutter
- 510: Kollimator
- 520: Einkoppelspiegel
- 530: Scannervorrichtung
- 540: Fokkusierlinse
- 550: Urunlenkspiegel
- 592: Shutter
- 594: Shutter
- 600: Stretcher
- 610: Gitter
- 620: Gitter
- 630: Spiegel
- 650: Linse
- 700: Kompressor
- 710: Gitter
- 720: Gitter
- 730: Spiegel
- 820: Linse
- 830: Kristallscheibe (Scheibenlaser)
- 840: Kühlvorrichtung (Scheibenlaser)
- 850: Pumplaserstrahl
- 860: Reflexionselement
- 870: ausgekoppelter Laserstrahl
- 880: Auskoppelspiegel
- A: Drehrichtung (Filter 210)
- D: Durchmesser des Pumplaserstrahls 850
- d: Dicke der Kristallscheibe 830
- L: gepulster Strahl aus dem Verstärker
- b: blauer Strahlanteil
- r: roter Strahlanteil

## Patentansprüche

1. Medizinisches Laserbehandlungsgerät, das zwischen einem cw-(continuous wave)-Betriebsmodus und einem Pulsbetriebsmodus schaltbar ausgebildet ist und mindestens eine erste Laserlichtquelle (410) zur Erzeugung eines cw-Laserstrahls umfaßt,
**dadurch gekennzeichnet, daß**
das Laserbehandlungsgerät ferner eine zweite Laserlichtquelle (480) zur Erzeugung eines gepulsten Laserstrahls umfaßt, die von mindestens einer der mindestens einen ersten Laserlichtquelle (410) gepumpt wird, und in dem cw-Betriebsmodus der cw-Laserstrahl mindestens einer der mindestens einen ersten Laserlichtquelle (410) und in dem Pulsbetriebsmodus der gepulste Laserstrahl der zweiten Laserlichtquelle (480) zur Behandlung verwendet wird, wobei das Laserbehandlungsgerät so ausgelegt ist, daß die zweite Laserlichtquelle (480) kontinuierlich auch im cw-Betriebsmodus von mindestens einer der mindestens einen ersten Laserlichtquelle (410) gepumpt wird.

2. Medizinisches Laserbehandlungsgerät nach Anspruch 1, **dadurch gekennzeichnet, daß** die mindestens eine erste Laserlichtquelle (410) einen Festkörperlaser umfaßt.

3. Medizinisches Laserbehandlungsgerät nach Anspruch 1 oder 2, **dadurch gekennzeichnet, daß** die mindestens eine erste Laserlichtquelle (410) mindestens eine Laserdiode umfaßt.

4. Medizinisches Laserbehandlungsgerät nach einem der Ansprüche 1 bis 3, **dadurch gekennzeichnet, daß** die zweite Laserlichtquelle (480) einen Scheibenlaser umfaßt.

5. Medizinisches Laserbehandlungsgerät nach einem der vorhergehenden Ansprüche, **dadurch gekennzeichnet, daß** die zweite Laserlichtquelle (480) so ausgelegt ist, daß sie zwischen unterschiedlichen Pulsbetriebsmodi schaltbar ist, in denen sie ein Laserlicht mit unterschiedlichen Laserpulsdauern und/oder unterschiedlichen Pulsfolgefrequenzen emittiert.

6. Medizinisches Laserbehandlungsgerät nach einem der vorhergehenden Ansprüche, **dadurch gekennzeichnet, daß** die zweite Laserlichtquelle (480) so ausgebildet ist, daß gepulstes Laserlicht mit einer Pulsdauer t abstrahlbar ist, wobei 20 ps ≤ t ≤ 500 ps ist.

7. Medizinisches Laserbehandlungsgerät nach einem der vorhergehenden Ansprüche, **dadurch gekennzeichnet, daß** die zweite Laserlichtquelle (480) so ausgebildet ist, daß gepulstes Laserlicht mit einer Pulsdauer t abstrahlbar ist, wobei t ≤ 1 ps ist.

8. Medizinisches Laserbehandlungsgerät nach einem der vorhergehenden Ansprüche 1 bis 5, **dadurch gekennzeichnet, daß** die zweite Laserlichtquelle (480) so ausgebildet ist, daß gepulstes Laserlicht mit einer Pulsdauer t abstrahlbar ist, wobei t = 5 bis 500 fs ist.

9. Medizinisches Laserbehandlungsgerät nach einem der vorhergehenden Ansprüche, **dadurch gekennzeichnet, daß** die zweite Laserlichtquelle (480) so ausgebildet ist, daß gepulstes Laserlicht mit einer Pulsdauer t abstrahlbar ist, wobei t ≥ 1 ns ist.

10. Medizinisches Laserbehandlungsgerät nach einem der vorhergehenden Ansprüche, **dadurch gekennzeichnet, daß** es so ausgestaltet ist, daß Licht mindestens einer Wellenlänge λ emittierbar ist, wobei λ zwischen 750 nm 1.100 nm liegt.

11. Medizinisches Laserbehandlungsgerät nach Anspruch 10, **dadurch gekennzeichnet, daß** eine erste Laserlichtquelle (410) so ausgebildet ist, daß sie eine erste Wellenlänge λ₁ für einen cw-Betriebsmodus emittiert, wobei λ₁ in einem Wellenlängenbereich von 750 nm bis 1.100 nm liegt.

12. Medizinisches Laserbehandlungsgerät nach Anspruch 10 oder 11, **dadurch gekennzeichnet, daß** die zweite Laserlichtquelle (480) so ausgebildet ist, daß sie eine zweite Wellenlänge λ₂ für einen Pulsbetriebsmodus emittiert, wobei λ₂ zwischen 750 nm und 800 nm liegt.

13. Medizinisches Laserbehandlungsgerät nach einem der vorhergehenden Ansprüche, **dadurch gekennzeichnet, daß** die zweite Laserlichtquelle (480) einen Yb:KGW-Laser, ein Yb:KYW-Laser, einen Yb:YAG-Laser, einen Nd: YAG-Laser oder einen Nd: YLF-Laser umfaßt.

14. Medizinisches Laserbehandlungsgerät nach einem der vorhergehenden Ansprüche, **dadurch gekennzeichnet, daß** es eine Steuervorrichtung und/oder eine Schaltvorrichtung zur Wahl der Betriebsmodi und/oder anderer Funktionen des medizinischen Laserbehandlungsgeräts umfaßt.

15. Medizinisches Laserbehandlungsgerät nach einem der vorhergehenden Ansprüche, **dadurch gekennzeichnet, daß** es ferner eine Vorrichtung (340) zur Erzeugung und/oder Steuerung eines sichtbaren Hilfs-Laserstrahls umfaßt.

16. Medizinisches Laserbehandlungsgerät nach einem der vorhergehenden Ansprüche, **dadurch gekennzeichnet, daß** es ferner mindestens ein Mittel zur Veränderung der Pulsdauer des von der zweiten Laserlichtquelle (480) emittierten Laserstrahls umfaßt.

17. Medizinisches Laserbehandlungsgerät nach einem der vorhergehenden Ansprüche, **dadurch gekennzeichnet, daß** es ferner einen Strahlteiler (440) umfaßt, der einen Teil der emittierten Leistung des mindestens einen ersten Laserlichtquelle (410) auskoppelt.

18. Medizinisches Laserbehandlungsgerät nach einem der vorhergehenden Ansprüche, **dadurch gekennzeichnet, daß** es steuerbare Abschirmmittel (492, 592, 594) aufweist, mit denen cw-Laserlicht mindestens einer ersten Laserlichtquelle (410) und/oder gepulstes Laserlicht der zweiten Laserlichquelle (480) abschirmbar ist.

## Claims

1. A medical laser treatment appliance which is designed to be switchable between a cw (continuous wave) operating mode and a pulsed operating mode and has at least one first laser light source (410) for producing a cw laser beam,
**characterized in that**
the laser treatment appliance furthermore has a second laser light source (480) for producing a pulsed laser beam, which is pumped by at least one of the at least one first laser light sources (410), and in which cw operating mode the cw laser beam from at least one of the at least one first laser light sources (410) is used for the treatment, and, in the pulsed operating mode, the pulsed laser beam from the second laser light source (480) is used for the treatment, with the laser treatment appliance being designed such that the second laser light source (480) is pumped continuously, even in the cw operating mode, by at least one of the at least one first laser light sources (410).

2. The medical laser treatment appliance as claimed in claim 1, **characterized in that** the at least one first laser light source (410) comprises a solid state laser.

3. The medical laser treatment appliance as claimed in claim 1 or 2, **characterized in that** the at least one first laser light source (410) comprises at least one laser diode.

4. The medical laser treatment appliance as claimed in one of claims 1 to 3, **characterized in that** the second laser light source (480) comprises a wafer laser.

5. The medical laser treatment appliance as claimed in one of the preceding claims, **characterized in that** the second laser light source (480) is designed such that it can be switched between different pulsed operating modes, in which it emits laser light with different laser pulse durations and/or at different pulse repetition frequencies.

6. The medical laser treatment appliance as claimed in one of the preceding claims, **characterized in that** the second laser light source (480) is designed such that pulsed laser light can be emitted with a pulse duration t, where 20 ps ≤ t ≤ 500 ps.

7. The medical laser treatment appliance as claimed in one of the preceding claims, **characterized in that** the second laser light source (480) is designed such that pulsed laser light can be emitted with a pulse duration t, where t ≤ 1 ps.

8. The medical laser treatment appliance as claimed in one of the preceding claims 1 to 5, **characterized in that** the second laser light source (480) is designed such that pulsed laser light can be emitted with a pulse duration t, where t = 5 to 500 fs.

9. The medical laser treatment appliance as claimed in one of the preceding claims, **characterized in that** the second laser light source (480) is designed such that pulsed laser light can be emitted with a pulse duration t, where t ≥ 1 ns.

10. The medical laser treatment appliance as claimed in one of the preceding claims, **characterized in that** the appliance is designed such that light can be emitted at at least one wavelength λ, where λ is between 750 nm and 1.100 nm. 10, **characterized in that** one

11. The medical laser treatment appliance as claimed in claim 10, **characterized in that** one first laser light source (410) is designed such that it emits a first wavelength λ₁ for a cw operating mode, where λ₁ is in a wavelength range from 750 nm to 1100 nm.

12. The medical laser treatment appliance as claimed in claim 10 or 11, **characterized in that** the second laser light source (480) is designed such that it emits a second wavelength λ₂ for a pulsed operating mode, where λ₂ is between 750 nm and 800 nm.

13. The medical laser treatment appliance as claimed in one of the preceding claims, **characterized in that** the second laser light source (480) comprises a Yb:KGW laser, a Yb:KYW laser, a Yb:YAG laser, an Nd: YAG laser or an Nd: YLF laser.

14. The medical laser treatment appliance as claimed in one of the preceding claims, **characterized in that** said appliance comprises a control apparatus and/or a switching apparatus for selecting the operating modes and/or other functions of the medical laser treatment appliance.

15. The medical laser treatment appliance as claimed in one of the preceding claims, **characterized in that** said appliance furthermore comprises an apparatus (340) for producing and/or controlling a visible auxiliary laser beam.

16. The medical laser treatment appliance as claimed in one of the preceding claims, **characterized in that** said appliance furthermore comprises at least one means for changing the pulse duration of the laser beam which is emitted from the second laser light source (480).

17. The medical laser treatment appliance as claimed in one of the preceding claims, **characterized in that** said appliance furthermore comprises a beam splitter (440) which outputs a portion of the emitted power of the at least one first laser light source (410).

18. The medical laser treatment appliance as claimed in one of the preceding claims, **characterized in that** said appliance has controllable shielding means (492, 592, 594), by means of which cw laser light from at least one first laser light source (410) and/or pulsed laser light from the second laser light source (480) can be shielded.

## Revendications

1. Appareil médical de traitement au laser, réalisé de façon à pouvoir être commuté entre un mode de fonctionnement cw (Continuous Wave) et un mode de fonctionnement pulsatoire et comprenant au moins une première source de lumière laser (410) pour produire un rayon laser cw, **caractérisé en ce que**
l'appareil de traitement au laser comprend en outre une deuxième source de lumière laser (480) pour produire un rayon laser pulsé, pompé par au moins une de la au moins une première source de lumière laser (410) et, au mode de fonctionnement cw, le rayon laser cw d'au moins l'une de la au moins une première source de lumière laser (410) et, au mode de fonctionnement pulsatoire, le rayon laser pulsé de la deuxième source de lumière laser (480) étant utilisé pour un traitement, l'appareil de traitement au laser étant conçu de manière que la deuxième source de lumière laser (480) soit pompée en continu, également en mode de fonctionnement cw, par au moins une de la au moins une première source de lumière laser (410).

2. Appareil médical de traitement au laser selon la revendication 1, **caractérisé en ce que** la au moins une première source de lumière laser (410) comprend un laser à semi-conducteur.

3. Appareil médical de traitement au laser selon la revendication 1 ou 2, **caractérisé en ce que** la au moins une première source de lumière laser (410) comprend au moins une diode laser.

4. Appareil médical de traitement au laser selon l'une des revendications 1 à 3, **caractérisé en ce que** la deuxième source de lumière laser (480) comprend un laser à disque.

5. Appareil médical de traitement au laser selon l'une des revendications précédentes, **caractérisé en ce que** la deuxième source de lumière laser (480) est conçue de manière à pouvoir commuter entre des modes de fonctionnement pulsatoire différents, auxquels elle émet une lumière laser avec des durées d'impulsion de laser différentes et/ou avec des fréquences de suites d'impulsions différentes.

6. Appareil médical de traitement au laser selon l'une des revendications précédentes, **caractérisé en ce que** la deuxième source de lumière laser (480) est réalisée de manière qu'une lumière laser pulsée, d'une durée d'impulsion t, puisse être irradiée, sachant que 20 ps ≤ t ≤ 500≤ ps.

7. Appareil médical de traitement au laser selon l'une des revendications précédentes, **caractérisé en ce que** la deuxième source de lumière laser (480) est réalisée de manière qu'une lumière laser pulsée puisse être irradiée, d'une durée d'impulsion t, sachant t ≤ 1 ps.

8. Appareil médical de traitement au laser selon l'une des revendications 1 à 5 précédentes, **caractérisé en ce que** la deuxième source de lumière laser (480) est telle qu'une lumière laser pulsée puisse être irradiée, d'une durée d'impulsion t, sachant que t = 5 à 500 fs.

9. Appareil médical de traitement au laser selon l'une des revendications précédentes, **caractérisé en ce que** la deuxième source de lumière laser (480) est réalisée de manière qu'une lumière laser pulsée puisse être irradiée, d'une durée d'impulsion t, sachant que t ≥ 1 ns.

10. Appareil médical de traitement au laser selon la revendication précédente, **caractérisé en ce qu'**il est conçu de manière à pouvoir émettre une lumière d'au moins une longueur d'onde λ, λ étant situé entre 750 nm et 1.100 nm.

11. Appareil médical de traitement au laser selon la revendication 10, caractérisé en ce la première source de lumière laser (410) est réalisée de manière qu' elle émette une première longueur λ₁ pour un mode de fonctionnement cw, λ₁ étant situé dans une plage de longueurs d'ondes de 750 nm à 1.100 nm.

12. Appareil médical de traitement au laser selon la revendication 10 ou 11, **caractérisé en ce que** la deuxième source de lumière laser (480) est réalisée de manière à émettre une deuxième longueur d'onde λ₂ pour un mode de fonctionnement pulsé, λ₂ étant situé entre 750 nm et 800 nm.

13. Appareil médical de traitement au laser selon l'une des revendications précédentes, **caractérisé en ce que** la deuxième source de lumière laser (480) est un laser Yb:KGW, un laser Yb:KYW, un laser Yb:YAG; un laser Nd:YAG, ou un laser Nd:YAF.

14. Appareil médical de traitement au laser selon l'une des revendications précédentes, **caractérisé en ce qu'**il comprend un dispositif de commande et/ou un dispositif de commutation, pour sélectionner les modes de fonctionnement et/ou d'autres fonctions de l'appareil médical de traitement au laser.

15. Appareil médical de traitement au laser selon l'une des revendications précédentes, **caractérisé en ce qu'**il comprend en outre un dispositif (340) pour produire et/ou commander un rayon laser auxiliaire visible.

16. Appareil médical de traitement au laser selon l'une des revendications précédentes, **caractérisé en ce qu'**il comprend en outre au moins un moyen de modification de la durée d'impulsion du rayon laser émis par la deuxième source de lumière laser (480).

17. Appareil médical de traitement au laser selon l'une des revendications précédentes, **caractérisé en ce qu'**il comprend en outre un diviseur de rayon (440) qui désaccouple une partie de la puissance émise, d'au moins une première source de lumière laser (410).

18. Appareil médical de traitement au laser selon l'une des revendications précédentes, **caractérisé en ce qu'**il présente des moyens d'écrantage (492, 592, 594) pouvant être commandés, à l'aide desquels la lumière laser cw d'au moins une première source de lumière laser (410) et/ou une lumière laser pulsée de la deuxième source de lumière laser (480) est susceptible d'être écrantée.
